(19) Europäisches Patentamt
European Patent Office
Office européen des brevets

(11) Publication number: 0 397 326 A1

# (12) EUROPEAN PATENT APPLICATION

(21) Application number: 90303958.4

(22) Date of filing: 11.04.90

(51) Int. Cl.5: C08K 5/12, C08L 27/06, A61L 33/00, A61J 1/00

(30) Priority: 11.04.89 IT 2010589
22.03.90 IT 1976090

(43) Date of publication of application:
14.11.90 Bulletin 90/46

(84) Designated Contracting States:
AT BE CH DE ES FR GB IT LI NL SE

(71) Applicant: BP Chemicals Limited
Belgrave House 76 Buckingham Palace Road
London, SW1W 0SU(GB)

Applicant: SISTEMI TERAPEUTICI S.P.A.
26020 Palazzo Pignano
Cremona(IT)

(72) Inventor: Clutterbuck, Paul Geoffrey
BP Chemicals Limited, Hayes Road
Sully, South Glamorgan CF6 2YU(GB)
Inventor: Boselli, Pietro M.
Sistemi Terapeutici S.P.A.
I-26020 Palazzo Pignano, Cremona(IT)

(74) Representative: Krishnan, Suryanarayana Kalyana et al
BP INTERNATIONAL LIMITED Patents & Agreements Division Chertsey Road
Sunbury-on-Thames Middlesex TW16 7LN(GB)

## (54) Plasticised polymer compositions.

(57) This invention relates to the use of tetraalkylpyromellitates as plasticisers for PVC for use in articles for biomedical use. The pyromellitates are less toxic than the conventionally used phthlates and trimellitates and are extracted less readily by the biological fluids in contact therewith. The articles that can be produced with PVC plasticised in this manner include dialysis tubes, catheters and blood bags.

EP 0 397 326 A1

## PLASTICISED POLYMER COMPOSITIONS

The present invention relates to plasticised polymer compositions, especially compositions based on polyvinyl chloride (hereafter "PVC"), comprising specific pyromellitate esters as plasticisers for the manufacture of articles for biomedical use and to articles made therefrom.

The use of polymeric materials have undoubtedly contributed to significant progress in medicine and surgery. For example, polymeric materials are used in haemodialysis, in devices for enteral and paraenteral nutrition, blood bags and the like.

Among the polymeric materials more commonly used are PVC, polyurethanes, silicones and the like. In the case of PVC, the plasticiser used hitherto has almost exclusively been diethyl hexyl phthalate (also known as dioctyl phthalate and hereafter "DOP"). More recently, for some uses, trioctyl trimellitate (hereafter "TOTM") has been recommended instead of DOP.

However, generally the use of polymers containing the ester plasticisers for biomedical applications has hitherto been unsatisfactory due to the susceptibility of the esters to extraction from the polymer plasticised therewith when in contact with biological fluids. Even if TOTM reduces such risk of being extracted from the polymer, there is nevertheless a risk of transfer of TOTM into the blood stream as a result of extraction, especially when articles made from polymers plasticised with TOTM e.g. bags, flow pipes etc, are in contact with blood.

It has thus been realised that for a plasticiser to be suitable for biomedical use, the articles containing them should be bio-compatible. That is, they should have the ability to be fully dispersed in the polymer plasticised therewith: they should be substantially free of the risk of being extracted by the biological fluids in contact therewith; and they should be capable of micro-compartmentalisation.

It has now been found that by appropriate choice of specific esters, the problems associated with prior art esters in biomedical use can be substantially mitigated.

Accordingly, the present invention is a plasticised polymer composition suitable for producing articles of biomedical use, characterised in that the plasticiser in the composition comprises one or more pyromellitate esters of the formula (I):

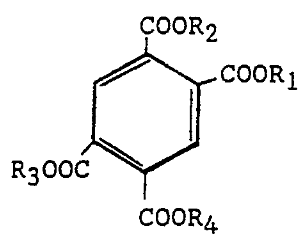

(I)

in which $R_1$, $R_2$, $R_3$ and $R_4$ are the same or different

(a) alkyl or alkenyl groups, which may be linear or branched, containing 1-15 carbon atoms, or,

(b) cyclic groups containing 5-6 carbon atoms in a ring, said ring being cycloaliphatic or aromatic with or without additional substituents in the ring structure.

Pyromellitates of formula (I) in which e.g. the substituents $R_1$-$R_4$ represent an alkyl substituent have previously been used as plasticisers for polymeric compositions only in the context of electrical uses but not in the context of biomedical use.

The pyromellitate ester plasticisers are preferably those in which groups $R_1$-$R_4$ are $C_1$-$C_{15}$ linear or branched alkyl groups. Amongst these, those in which at least one of the substituents $R_1$-$R_4$ represents an ethyl or an octyl group are most preferred.

A comparison of the relative risks of extraction of conventional plasticisers with the pyromellitate esters in the compositions of the present invention shows that when articles made from PVC containing these plasticisers are in contact e.g. with bovine blood, tetraoctyl pyromellitate is from 95-98% less extractable than DEHP or dioctyl phthalate, and is at least 85% less extractable than TOTM. Thus it is clear that in biomedical use, articles made from polymer compositions containing pyromellitate esters as plasticisers show the following advantages. They show:

1. lower toxcity than TOTM or dioctyl phthalate and hence do not adversely affect biological processes,

2. substantially neglible risk of extraction when in contact with blood or other biological fluids, and

3. improved plasticising efficiency and durability of articles made from polymers containing said

plasticisers.

The plasticisers can be used in the polymer compositions suitably in an amount of at least 30-100 parts per hundred of the resin (hereafter "phr"), preferably 60-90 phr based on the polymer in the composition, depending upon the polymer to be plasticised, the hardness or flexibility of the final product desired, or the barrier properties desirable for the specific medical/biomedical use.

The polymers in the polymer compositions are suitably thermoplastic polymers, especially PVC, although they can also be used advantageously in polyurethane and silicone polymers.

The polymer composition can be prepared e.g. by blending the plasticiser with the polymer. The blending is suitably carried out at elevated temperature from 60-150°C. The polymer compositions can contain in addition conventional internal or external lubricants, stabilisers and the like. Examples of stabiliser include zinc dioctanoate, zinc distearate, calcium distearate and mixtures of these, N,N′-diacylethylene diamines preferably having palmitoyl or stereoyl groups as acyl groups, epoxidised oils e.g. soyabean oil, linseed oil.

The polymer compositions can be shaped into an article for biomedical use suitably by extrusion e.g. into films. Where such films are used for producing blood bags, the films should be of a minimum thickness of about 0.35mm. Where the compositions are to be used for producing tubes, such tubes can be produced also by extrusion and the thickness of the tube wall can be adjusted as desired. Typically for use in dialysis the tube has an internal diameter of about 4.5-5mm and an external diameter of about 6-8mm i.e. a tube wall thickness of about 0.5-1.8mm.

The extrusion of the composition is suitably carried out at a temperature of 120-190°C.

The polymer compositions of the present invention containing the pyromellitate esters as plasticisers can be used to produce many articles for biomedical use such as, e.g., tubes, flexible tubes, bags, catheters, sheets, sheaths, flow-pipes and other such components which are needed for direct usage and/or as accessories for the functioning of any apparatus for biomedical uses including dialysis, enteral and paraenteral nutrition, extra-corporeal circulation, artificial organs and the like.

In another embodiment, the present invention relates to any shaped article produced from the polymer compositions claimed and described above.

The present invention is further illustrated with reference to the following Examples.

Examples:

Two types of dialysis tubes were produced by conventional extrusion techniques using different formulations. The formulations used are shown below:

| Compound | Trade Name/Source | Quantities Used (Kg) | |
|---|---|---|---|
| | | Tube 1 | Tube 2 |
| PVC | RAVINIL* S70M (Ex Enichem) | 50.000 | 52.000 |
| TOPM | BP Chemicals | 35.526 | 45.000 |
| Epoxidised Soya Oil | EDENOL-D82* (Ex Henkel Chimica) | 1.885 | 2.150 |
| Stearate Salts | S.T.88 from Zn/Ca Stearate | 0.153 | 0.174 |
| Ca/Zn Complex | PROSPER 128* (Ex Commer spa Lodi, Milan) | 0.048 | 0.054 |
| Glycerol Ester | IRGAWAX 361* (Ex Ciba Geigy) | 0.048 | 0.054 |
| Lubricant | VASELINE P.F. (Ex Witco - Snowwhite EC USP) | 0.026 | 0.030 |
| Notes: | | | |
| PVC - Polyvinyl Chloride<br>TOPM - Tetraoctyl Pyromellitate | | | |

* - Registered Trade Mark

S.T.88 - Made by intimately mixing calcium stearate (66%w/w) and zinc stearate (34%w/w) powders.

The blending of the formulation was achieved at a mixing, granulation and extrusion temperature as shown below:

| Stage/Technique | Temperature °C | |
|---|---|---|
| | Tube 1 | Tube 2 |
| Mixing: | | |
| Initial Plasticisation | 65 | 60 |
| Final | 110 | 105 |
| Granulation (Mixing at 20 rpm): | 20-170 | 23-170 |
| Extrusion (Head temperature): | 170-90 | 170-90 |

The resultant tubes had the following characteristics:

| Product Characteristic : | Tube 1 | Tube 2 |
|---|---|---|
| Internal Diameter (mm) | 4.7 | 4.7 |
| External Diameter (mm) | 7.2 | 7.2 |
| Wall Thickness (mm) | 1.25 | 1.25 |
| Shore Hardness | A 70 D35 | A 60 D35 |
| Tensile Strength (MPa) | 16.6 | 13.8 |
| Elongation at break (%) | 413 | 394 |

Further dialysis tubes of similar dimensions were produced using PVC formulations containing the plasticisers dioctyl phthalate (DOP) and trioctyl triellitate (TOTM) to compare the performance thereof with the tubes containing tetraoctyl pyromellitate (TOPM). The formulations used were similar to that for Tube 1 above but in each case the quantities of PVC and plasticiser used were:

| | |
|---|---|
| DOP - 45 kg | PVC - 75 kg |
| TOTM - 54 kg | PVC - 75 kg |
| TOPM - 35.526 kg | PVC - 50 kg |

The resultant tubes were tested for their loss of plasticisers by extraction when in contact with bovine blood circulated through each one off these tubes thermostated at 37°C over 4 hours at the rate of 15 minutes per cycle. The results showed that the rate of extraction of the plasticisers were as follows:
TOPM is extracted in an amount which is from 90-98% lower than DOP while TOPM is extracted in an amount which is from 70-80% lower than TOTM.

For the purpose of testing the extractability or partitioning of the plasticiser by the biological fluids, in particular by blood, one can assume that blood is substantially made up of two components: a hydrophilic component and lipophilic component in the proportions of 90:10. The tests were therefore carried out using pure plasticisers for determining the extractability or partitioning by shaking the plasticisers with a 50:50 w/w homogeneous hydrophilic (water)/lipophilic (olive oil) dispersion. In these experiments, which took the worst scenario (because of the relatively high lipophilic content thereof), the results showed that the extraction/partition of the respective plasticisers by the homogeneous dispersion was as follows:

TOPM is extracted/partitioned into the lipophilic phase in an amount which is from 75-85% lower than DOP while TOPM is extracted in the same phase in an amount which is from 55-65% lower than TOTM.

The extraction/partition of the plasticisers into the hydrophilic phase is relatively negligible.

The acute toxicity of these plasticisers to mice is known to be in the order DOP > TOTM > TOPM. Thus, in addition to being less toxic to the biological processes than the other two well known PVC plasticisers, these results show that TOPM is also extracted to a significantly lesser extent by the biological fluids. The extraction-toxicity synergism results are therefore even more favourable when using TOPM.

**Claims**

1. A plasticised polymer composition for producing shaped articles of biomedical use, characterised in that the plasticiser in the composition comprises one or more pyromellitate esters of the formula (I):

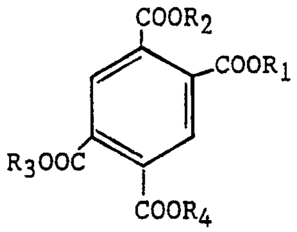

(I)

in which $R_1$, $R_2$, $R_3$ and $R_4$ are the same or different and represent

(a) alkyl or alkenyl groups, which may be linear or branched, containing 1-15 carbon atoms, or,

(b) cyclic groups containing 5-6 carbon atoms in a ring, said ring being cycloaliphatic or aromatic with or without additional substituents in the ring structure.

2. A polymer composition according to Claim 1 wherein at least one of the substituent groups $R_1$-$R_4$ represents an ethyl or an octyl group.

3. A polymer composition according to Claim 1 or 2 wherein the plasticiser is present in an amount of from 30-100 phr based on the polymer in the composition.

4. A polymer composition according to any one of the preceding Claims wherein the plasticiser is present in an amount from 60-90 phr based on the polymer in the composition.

5. A polymer composition according to any one of the preceding Claims wherein the polymer in said composition is a thermoplastic polymer.

6. A polymer composition according to any one of the preceding Claims wherein the polymer in the composition is PVC.

7. A polymer composition according to any one of the preceding Claims wherein said composition is used for producing articles of biomedical use, said articles being selected from films, tubes whether flexible or rigid, bags, catheters, sheets, sheaths, flow-pipes and artificial organs.

8. A polymer composition according to Claim 7 wherein said composition is a thermoplastic PVC composition used for producing films and/or blood bags.

9. A polymer composition according to Claim 7 or 8 wherein the film produced has a thickness of about 0.35mm.

10. Articles for biomedical use whenever produced from a plasticised polymer composition comprising one or more pyromellitate esters of formula (I) as plasticiser.

European Patent Office

# EUROPEAN SEARCH REPORT

Application Number

EP 90 30 3958

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-3218289 (S. ROWLAND)<br>* claims * | 1-9 | C08K5/12<br>C08L27/06<br>A61L33/00<br>A61J1/00 |
| X | EP-A-0178778 (EXXON RESEARCH AND ENGINEERING COMPANY)<br>* claims 4-5; examples * | 1-9 | |
| X | DE-A-3444155 (BIOTEST-SERUM-INSTITUT)<br>* page 7, line 22 - page 8, line 10; claim 1 * | 1-10 | |
| X,P<br>X | EP-A-0340305 (KAO CORPORATION)<br>* page 29, lines 14 - 15; claims 8, 9, 15 *<br>& WO-A-8805302 | 1-10 | |
| | | | TECHNICAL FIELDS SEARCHED (Int. Cl.5 ) |
| | | | C08K<br>C08L<br>A61J<br>A61L |

The present search report has been drawn up for all claims

| Place of search | Date of completion of the search | Examiner |
|---|---|---|
| THE HAGUE | 13 JULY 1990 | HOFFMANN K.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document



EPO FORM 1503 03.82 (P0401)